# EUROPEAN PATENT SPECIFICATION

(11) **EP 0 321 736 B1**
(45) Date of publication and mention of the grant of the patent: **01.06.1994**
(21) Application number: 88119780.0
(22) Date of filing: 28.11.1988
(51) Int. Cl.: G01N 33/53, B01L 3/00

(54) **Agglutination reaction device**
Vorrichtung zur Agglutinierungsreaktion
Dispositif pour réaction d'agglutination

(30) Priority: 23.12.1987 US 138253
(43) Date of publication of application: 28.06.1989
(73) Proprietor: ABBOTT LABORATORIES, Abbott Park, Illinois 60064-3500 (US)
(72) Inventor: Parsons, Robert G., Green Oak Illinois 60048 (US); Kowal, Robert, Libertyville Illinois 60048 (US); Houseman, Kenneth Russell, Racine Wisconsin 53403 (US); O'Connell, Michael B., Waukegan Illinois 60085 (US)
(74) Representative: Modiano, Guido, Dr.-Ing.

(56) References cited:
- EP-A- 0 010 456
- EP-A- 0 212 314
- EP-A- 0 215 419
- DE-A- 3 022 940
- DE-A- 3 438 245

## Description

### BACKGROUND OF THE INVENTION

The present invention is directed toward a device and method for performing an agglutination reaction of immunochemical particles. The agglutination reaction device is designed to provide a convenient means for performing and reading the results of an agglutination reaction.

Agglutination reactions and their procedures are generally well known in the art. A typical agglutination reaction consist of the clumping together in suspension of antigen-bearing cells, microorganisms, or particles in the presence of specific antibodies. This clumping or agglutination of particles is then monitored to determine the absence or presence of an analyte sought to be detected.

One method for reacting immunochemical particle reagents is by the placement of the liquid reagents on a glass slide and generally rocking or swirling the slide back and forth to cause the reagents to mix and form agglutinations. Methods have also been developed to avoid the necessary swirling of the particle reagents in order to visualize the agglutinations. For example, U.S. Patent 4,596,695 discloses an agglutination reaction chamber for reacting immunochemical particle reagents. The chamber includes a first transparent panel having a first surface and a second panel having a second surface spaced apart from the first surface to define a chamber between. The chamber intrinsically causes immunochemical particle reagents to react by capillary motion without an external motion imparted to the chamber.

EP-A-212 314 discloses a capillary flow device which is disclosed as being suitable for agglutination reactions. The capillary path may be varied to control mixing, incubating, and detection.

An object of the present invention is to provide a device that can be easily adapted for use in the automated diagnosis of a plurality of samples. Another object of the present invention is to provide a device capable of performing multiple, highly sensitive, diagnostic tests simultaneously on a single sample in a single device. In particular, the present invention is directed toward a device that can be used in an automated fashion where the reaction can be rapidly performed and monitored with a minimum of sample material.

### SUMMARY OF THE INVENTION

The present invention is directed toward an agglutination reaction chamber for performing agglutination immunoassay reactions. The device comprises a first hydrophilic surface and a parallel second surface wherein said first surface has channels such that when the first and second surface are brought into contact with each other an agglutination reaction chamber is formed for conducting fluid by capillary action. The agglutination reaction chamber can additionally include a sample receiving well contiguous with the ingress of the chamber formed by the first and second surfaces. Also, the chambers have means for controlling the flow of fluid and may have a predetermined amount of reagent dispersed in them for performing an assay.

In the agglutination reaction chamber of the present invention the reagent can be present in dried spots or strips. It is also possible to suspend the reagent in a water-soluble polymer.

The means of the invention for controlling the flow of fluid in the chambers comprises a water-soluble material, such as a water-soluble polymer, dried in portions of the channel. The water-soluble material can comprise polyvinylpyrrolidone, polyvinylalcohol, gelatin, or bovine serum albumin.

Supplementary means for controlling the flow of fluid can be the configuration of the channels or geometric formations within the channels such as ridges. Preferably, the ridges are formed in the channels which extend across the entire width of the channels and for at least a portion of the length of the channel.

The agglutination reaction chamber is constructed to be very small in size in order to accommodate automated and efficient use of sample and reagents. Preferably, the length of a chamber is from 10 to 75 mm, the channels are from 0.01 to 5.0 millimeters in depth and from 0.1 to 10.0 millimeters in width. A typical size for an entire agglutination reaction device having four chambers and a sample receiving well is 37.5 mm x 12.5 mm x 1.5 mm (1 x w x h).

### BRIEF DESCRIPTION OF THE DRAWINGS

Figure 1 is a top perspective view of one embodiment of the present agglutination reaction device showing a sample receiving well and four reaction chambers without the flow controlling means of the invention shown in the terminal region.

Figure 2 is a top perspective view of one embodiment of a bottom surface of the present agglutination reaction device showing a sample receiving well and four channels as well as the flow controlling means of the invention shown in the terminal region.

Figure 3 is a top perspective view of another embodiment of a bottom surface of the present agglutination reaction device showing a sample receiving well and four channels with supplementary flow controlling means (ridges) formed therein without the flow controlling means of the invention shown in the terminal region.

Figure 4 is a cross-sectional view of a channel shown in Fig. 3 showing one embodiment of a ridge formed in a channel to control the flow of fluid.

Figure 5 is a cross-sectional view of a channel shown in Fig. 3 showing another embodiment of a ridge formed in a channel to control the flow of fluid.

### DETAILED DESCRIPTION OF THE INVENTION

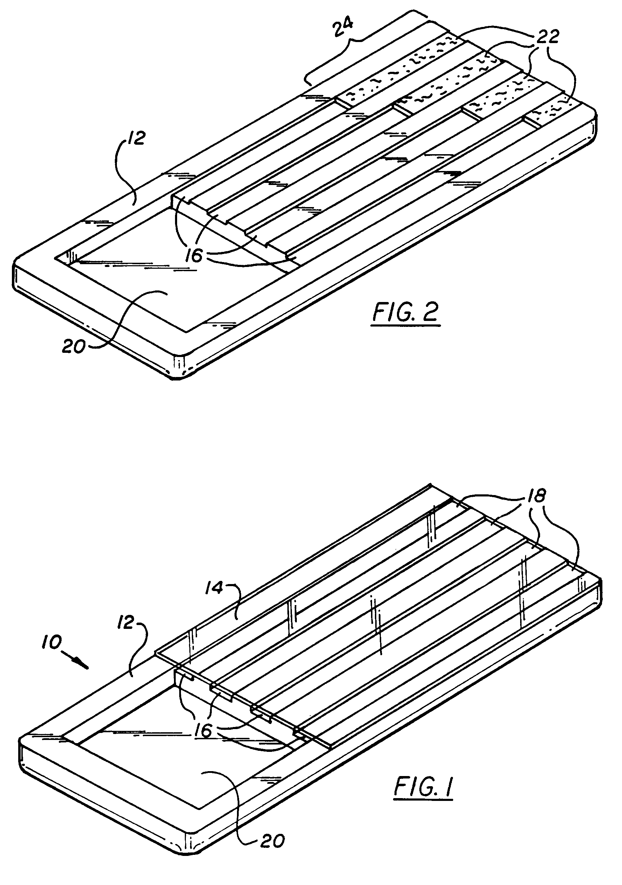

The present invention is directed toward a device and method suitable for performing agglutination reactions. One such embodiment is shown in Figures 1 and 2 in combination. The means of the invention for controlling the flow of fluid in the device of the invention is illustrated in Figure 2. The device 10 as shown in Fig. 1 generally comprises a first surface 12 and a parallel second surface 14 one of which has channels 16 formed therein such that when the two surfaces are placed together a chamber or microchamber 18 is formed through which liquid will flow by capillary action. In order to have capillary flow it is required that the surface having the channels formed therein is hydrophilic. Preferably, the surfaces are designed such that they create a plurality of chambers or microchambers 18 when assembled.

The surface having channels grooved therein also preferably has a sample receiving well 20 which is open to each of the channels 16. The receiving well 20 is positioned such that it is not covered by the second surface 14 as shown in Fig. 1. A sample can then be placed in the receiving well 20, such that it can be wicked into the chambers 18 by capillary action. Thus it is possible to form a device having multiple chambers and one large sample receiving well such that a single drop of sample can be placed in the well and wicked into the multiple chambers. Each chamber can then perform a different agglutination assay on a single sample.

It is a further objective of the present invention to provide a device, which is small enough to be utilized in automated instrumentation and monitored by automated means for the presence of agglutinated particles in each of the chambers. Thus in a preferred embodiment of the agglutination device, the lower surface 12 will be opaque and the upper surface 14 will be transparent to transmitted or reflected light where an optical scanner can be positioned above the device 10. Thus, the opaque lower surface 12 gives a better background for detection of agglutination particles.

For example, when a solution of cells is introduced to one end of a chamber 18, containing antibodies directed against antigens on the cells and dried onto the interior of the channels 16, the solution will migrate through the channel mix with the antisera and the cells will aggregate. This will all occur without any centrifugation or mixing steps. Control of the flow of the liquid through the channel is necessary because the agglutination reaction occurs preferably during the period of liquid flow. Sufficient incubation time must be incorporated into the period of liquid flow to achieve maximal reactions.

Means for controlling the flow of sample through the capillary chambers 18 in order to properly perform the agglutination reaction are also provided. The method of the invention is to place a water-soluble material, such as a water-soluble polymer, in at least a portion of the channels that will be resolubilized by the application of a sample material. Examples of water-soluble substances can include polyvinylpyrrolidone (PVP), gelatin, polyvinyl alcohol, or bovine serum albumin.

Liquid migration through the chambers 18 of the agglutination reaction device 10 can be controlled through the use of small spots or stripes of dried water-soluble material placed in the channels 16. As shown in Fig. 2, a water-soluble material 22 is dried in the channels 16 at their terminal region 24. These spots or strips of dried substance 22 can be of varying length depending upon the amount of flow control desired. When spots of a rapidly rehydrating material is dried along a chamber, the liquid will migrate through the chamber rapidly. When spots of a slowly hydrating material is dried along a chamber, the liquid will migrate through the chamber much more slowly. By using spots with different chemical make-up and different rehydration properties, the liquid flow through the channel can be controlled to include areas of rapid flow and areas of slow flow. By employing dried materials that rapidly rehydrate and slowly rehydrate in the same chamber, it is possible to create channels with predetermined incubation times at various zones throughout the chamber.

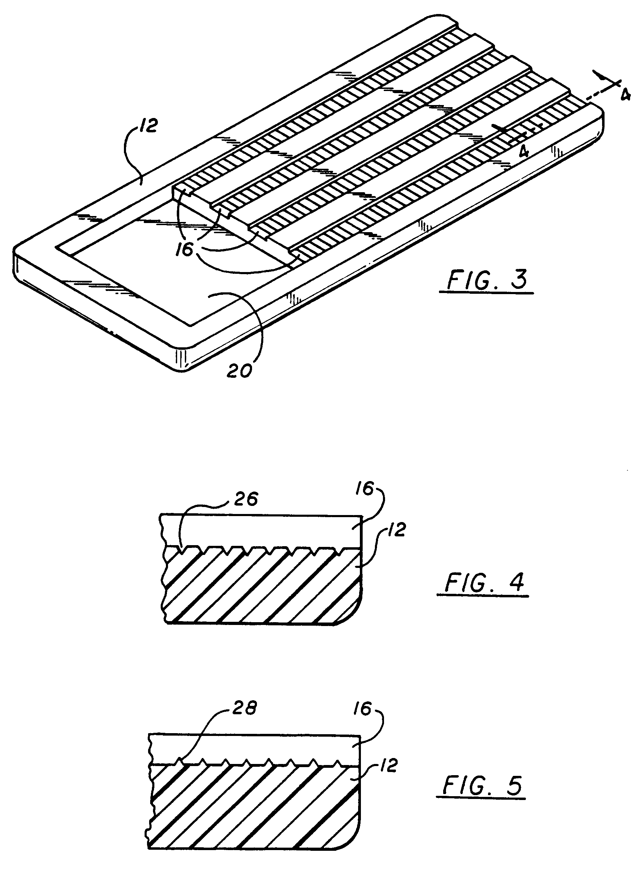

Supplementary means for controlling the flow of fluid are provided by the geometric configuration of the chamber and or the internal shape of the chambers as depicted in Figure 3-5. Preferably, ridges 26 or 28 (shown in the breakaway sectional views Figs. 4 and 5) are constructed in each of the channels 16 which extend across the width of the channel 16 and extend for at least a portion of the length of the channel 16. More preferably, the entire length of the channel 16 is prepared to have ridges 26 or 28. These ridges influence the movement of the liquid along the channel 16 and evenly distribute the sample through the channel 16.

All types of agglutination-based assays can be accommodated with the subject device. In some instances, a soluble reagent can be dried as spots or strips in the chamber, for example, in blood typing. In other instances, the particulate reagent, such as a latex reagent, can be dried in the chamber. In yet another approach, a reagent can be dispersed in a solution which is placed in the chamber. One preferred reagent solution is microparticulates in a solution of dextran and sucrose. Preferably, the microparticulate reagent is mixed in a solution of 2.5 to 5.0 percent by weight dextran and from 15 to 20 percent by weight sucrose. Another preferred solution for mixing reagents is FICOLL (a trademark by Sigma Chemical Co., St. Louis, MO for a nonionic synthetic polymer of sucrose) from 20 to 30 percent by weight. Also, depending on the requirements of the assay, the flow of the liquid through the chamber can be controlled as described above to accommodate any necessary incubation times and assay sequences.

A particularly unique feature of the subject device is the ability to simultaneously perform multiple assays while utilizing a very small amount of sample material, for instance, a single drop. Also, the agglutination assay is essentially self-performing once the drop has been added to the agglutination reaction device.

The device is especially suitable for use in an automated fashion where the agglutination reaction can be monitored by an optical scanner. For example, the construction of the agglutination reaction device enables one to use an image analysis system available from Olympus (CUE-2, Lake Success, N.Y.) to determine the quantity and concentration of agglutinated material. The agglutination reaction device is illuminated, such that transmitted or reflected light can be read by the reader. The image is then computer analyzed to determine the quantity of agglutination which has occurred and to enhance the image for very accurate and sensitive determinations. By confining the sample to a chamber such as formed in the agglutination reaction device, there is no problem with curvatures of droplets or water which could interfere with the image seen by the reader. Thus, the uniformity of the reacted sample and reagents achieved by the agglutination reaction device provides an excellent imaging format for a reader or other imaging devices. Besides being able to read the transmission of light through the bottom of the agglutination reaction device, it is also possible to read reflected light because the sample and reacted reagents are confined to capillary chambers formed by the agglutination reaction device.

Again referring to Figures 1 and 2 in combination, one embodiment of the present invention is shown. Figure 1 shows a perspective view of device 10 constructed of two layers of material, a bottom layer 12 covered by a top layer 14. Layer 12 has a plurality of channels 16 and a sample well 20 formed into the surface, The sample well 20 is contiguous with the ingress of each of the chambers 16. Each of the chambers 18 can have ridges 26 or 28 such as depicted in Figs. 4 and 5 which extend for the entire length of the channel 16. The ridges 26 or 28 influence fluid to smoothly flow from sample well 20 throughout the chamber 18.

In the construction of the device 10, either the bottom layer 12 or the upper layer 14 can be opaque. Preferably the layer which is further from an optical scanning device is opaque to enhance the background. It is required that the bottom surface 12 be hydrophilic or wettable such that the capillary flow is induced when a sample is placed in contact with the ingress of a chamber 18. This is accomplished by using a hydrophilic material for the surface 12; however, it is also possible to chemically treat or coat an otherwise hydrophobic materials such that they are hydrophilic. This preparation of a hydrophilic surface can also be used to influence the flow rate in the capillary chamber 18 so formed.

Suitable materials for preparing layer 12 as shown in Figs. 2 and 3 are cellulose acetate butyrate. In a preferred embodiment, the agglutination reaction device is prepared by molding a layer of cellulose acetate butyrate (CAB), commercially available from Eastman Chem. Prod. Inc., Kingsport, TN, to have a plurality of channels 16 from 0.010 to 5 millimeters in depth and from 0.1 to 10 millimeters in width. Each of the channels 16 extends from a larger well 20 molded into one end of the layer 12 of CAB. Next, a piece of transparent tape 14 sufficient to cover all the channels 16 molded into the CAB is applied to the surface 12 to form the capillary chambers 18. A section of adhesive cellophane tape can be used to provide the upper cover or surface for the recessed surface 14 to form the capillary chambers 18. Other hydrophobic materials can be used to form the upper surface 14 of the chambers 18.

The preferred overall dimensions of the agglutination reaction device is from 10 to 75 mm in length, from 5 to 20 mm in width and from 0.5 to 5.0 mm thick. The dimensions of the channels in the hydrophilic surface are preferably from 0.01 to 5.0 mm in depth and from 0.1 to 10.0 mm in width.

The very small size of the reaction device allows for the rapid and convenient handling of a plurality of devices and therefore samples. The device can then be loaded into an automated apparatus which indexes and scans the individual channels for the assay result and records this information for future access. The small dimensions of the agglutination reaction device also provide for efficient use of sample and reagents.

The following examples are provided to further demonstrate the agglutination reaction device of the subject invention.

### EXAMPLE 1

A steel mold was made that could be used to injection mold plastic devices of the general structure shown in Figure 1. The external dimensions of these pieces were 38 mm x 12.7 mm x 1.5 mm (1 x w x h). This design yielded pieces having one large sample receiving cavity or sample receiving well at one end of the part and 4 shallow channels that extend from the well to the opposite end of the part as shown in Fig. 1. These channels have flat bottoms and have dimensions of 25.4 mm x 1.5 mm x 0.075 mm (1 x w x d). This design of the device, having channels with flat bottoms is called Type I. Additional molds were made with identical designs as Type I except that the channels had the additional feature of ridges or grooves extending laterally across the bottoms of the channels (Types II and III - as shown in Figs. 3, 4 and 5). For Types II and III pieces, each channel has 39 individual features spaced 0.6 mm apart.
Several parts were obtained from these molds using the resin cellulose acetate butyrate (Eastman Chemical Products, Inc. Kingsport, TN). Molded pieces were washed for 90 minutes in KOH at 45°C, rinsed extensively with distilled water, and then allowed to air dry. These parts were overlaid with 1" wide transparent tape (No. 5910, 3M Co., St. Paul, MN) to completely cover the channels and overlap slightly the larger cavity sample receiving as shown in Fig. 1. Taping in this manner permitted the tape to adhere to the ridges between each of the channels, thus isolating each channel from the others. When a drop of water or other liquid solution was added to the sample receiving cavity of these taped parts, it would immediately flow through each of the channels by capillary action within a time range of 1-2 seconds.

### EXAMPLE 2

To control the rate of fluid flow through the channels of these devices, polyvinylpyrrolidone (PVP) solutions were dried into the channels. Solutions of PVP (Sigma Chemical Co., #PVP-360, St. Louis, MO) were prepared in distilled water at final concentrations of 2, 3, 4, and 5% (w/v). Small volumes (1.5 microliters) of these solutions were applied into each channel of KOH-washed chips of all three types described in Example 1. The PVP solutions were spread evenly over the distal half of the channel, farthest from the sample cavity. Following the drying of the pieces at 45°C for 10 min., tape was applied as described in Example 1. Distilled water was applied to the sample cavity of all chips and the time for the liquid to migrate to the end of each channel was monitored. The results as shown in Table 1 demonstrate that increases in the concentration of PVP dried in the channels results in dramatic increases in the time (measured in seconds) it takes for the distilled water to flow in the channel. Differences in the geometries of the channel designs (Type I vs. Type II vs. Type III) also produce different flow rates even with identical amounts of PVP in the channels.

**TABLE 1**

| Effects of PVP and Channel Design on Flow Rate (seconds) | | | |
|---|---|---|---|
| PVP Conc. | Type I | Type II | Type III |
| 2% | 7 | 151 | 97 |
| 3% | 190 | 344 | 385 |
| 4% | 438 | 690 | 723 |
| 5% | 791 | 1371 | 853 |

### EXAMPLE 3

Type I pieces were prepared as described in Example 2 with 1.5 µl volumes of 3% PVP. Prior to taping of the pieces, 0.5 µl volumes of Blood Grouping antisera (anti-A or anti-B (Dade, Miami, FL) diluted 1/4 in 10% sucrose) were applied in the proximal portion of each channel. The antisera was allowed to dry for 10 minutes in a 45°C incubator, and the pieces were then taped as described in Example 1.
Solutions of human erythrocytes (10% v/v) of types A1, A2, B, and O (Gamma, Houston, TX) were added to the central cavities of these pieces. In each case the blood cell suspension would flow half way down each channel very rapidly (1-2 seconds) and then very slowly (7-10 minutes). During the period of slow flow, cells moving through channels with their homologous antisera (Type A1 or A2 cells with anti-A, or Type B cells with anti-B) would aggregate into clumps which were plainly visible. Cells moving through channels with non-homologous antisera (Type A1, A2, or O with anti-B, or Type B or O with anti-A) did not aggregate but remained in a uniform suspension.

### EXAMPLE 4

Fixed human erythrocytes, Duractyes® (Abbott Laboratories, N. Chicago, IL) were coated with an antigen preparation of HTLV I virus. These cells were suspended in phosphate buffer at a 10% concentration (v/v). Serum samples (20 µl) were mixed with 10 µl aliquots of these DURACYTE® and then the solution was immediately added to the central cavity of Type II pieces which had been loaded with 1.5 µl of 3% PVP and taped as described in Example 2. Similar to Example 3, the solution flowed rapidly through the first half of each channel and then much more slowly through the later portion. When serum samples containing antibodies to HTLV I were used in these tests, the cells would aggregate during the period of slow flow, and such aggregates could be visualized directly or through the use of slight magnification (approximately 10 X) with a dissecting microscope, hand magnifier, or other such device. Samples that do not contain antibodies to HTLV I do not cause aggregation of the cells. The agglutination or aggregation of the cells occurs during the period of fluid flow through the channel and it stops when the fluid reaches the end of the channel. For these types of tests, the reactions take approximately 10 minutes to run to completion, and the final reaction pattern is stable for at least one hour without change.

### EXAMPLE 5

DURACYTE® were coated with affinity purified goat antibodies directed against Hepatitis B surface antigen at a final concentration of 100 µg/ml in the presence of 0.05% chromic chloride in 0.1 M acetate buffer pH 4.0. These cells were overcoated with 1% bovine serum albumin (Sigma Chemical Co., St. Louis, MO) in 25 mM Tris-HCl (pH 7.4) buffer and then resuspended with 0.1% bovine serum albumin in phosphate buffered saline (pH 7.4) containing 5% normal goat serum at a final cell concentration of 10% (v/v). These cells were reacted with serum samples as described in Example 4. Aggregation of the DURACYTE® was visible with sera having HBsAg at concentrations of 1 ng/ml or greater within approximately 10 minutes.

### EXAMPLE 6

DURACYTE® coated with anti-HBsAg and described in Example 5 were reacted with sera containing 0, 1, 3.1, 6.3, or 12.5 ng/ml of HBsAg as described in Example 4. Ten minutes after the samples and cells were mixed, the pieces were placed on a microscope (Model SZH, Olympus Corp., Lake Success, NY) which was connected to an image analyser system (Olympus Corp., Lake Success, NY). The image analyzer was set to detect an area within the central region of an individual channel. The image of the DURACYTE® mixture within the channel was analyzed to reveal the areas of aggregates. Aggregates were detected as the darker, more dense regions in the image as compared to non-aggregated DURACYTE® The percent of the area in a given window which was occupied by aggregates was calculated to give a numerical estimate of the degree of the reaction. As seen in Table 2, the percent area of the aggregates was directly proportional to the amount of HBsAg in the sample.

**TABLE 2**

| HBsAg Conc. | Percent Area |
|---|---|
| 0 | 0.04 |
| 1 | 0.45 |
| 3.1 | 1.62 |
| 6.3 | 3.87 |
| 12.5 | 6.32 |

### EXAMPLE 7

DURACYTE® coated with anti-HBsAg as described in Example 5 were suspended in a solution of 20% sucrose and 2.5% dextran - 77,800 MW (Sigma Chemical Co., St. Louis, MO) at a final concentration of 10% (v/v). One microliter aliquots of this DURACYTE® suspension was applied to the proximal portions of channels of Type II pieces which had been precoated with 1.5 µl of 3% PVP in the distal portion of the channel as described in Figure 5. The pieces were dried at 45°C for 10 min. and then taped as described in Example 1. Thirty microliter aliquots of specimens containing 0, 6.1, or 25 ng/ml HBsAg were added to the sample wells of pieces with dried DURACYTE® reagents. As the sample flowed through the channels, the DURACYTE® were rehydrated and flowed to form a fairly uniform suspension for the sample without HBsAg. Samples containing HBsAg revealed aggregation of the cells as seen in previous Examples. The time for these assays to come to completion was approximately 10 minutes.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. A device for performing agglutination reactions, said device comprising a first hydrophilic surface and a parallel second surface, wherein said first surface comprises at least one channel forming means therein such that, when said first and second surfaces are brought into contact with each other, at least one agglutination reaction chamber is formed for conducting fluid by capillary action at a chamber induced flow rate, said chamber being defined along its length by said second surface and said channel forming means, at least one of said first and second surfaces comprising at least one portion of sufficient transparency to permit the results of an agglutination reaction to be detected, said agglutination reaction chamber comprising means for controlling the rate of fluid flow within said chamber comprising a water-soluble material dried within a portion of said chamber distal to the fluid receiving end, wherein said controlling means induces a second reduced fluid flow rate sufficient to permit an agglutination reaction to occur.

2. The device of Claim 1 wherein said water-soluble material is polyvinylpyrrolidone, polyvinylalcohol, gelatin or bovine serum albumin.

3. The device of Claim 1 or 2 further comprising a predetermined amount of reagent for performing an assay disposed within said reaction chamber and prior to said water-soluble material controlling the rate of fluid flow.

4. The device of Claim 3 wherein said reagent is disposed as dried spots or strips.

5. The device of Claim 3 wherein said reagent comprises microparticles.

6. The device of Claim 3 wherein said reagent is suspended in a water-soluble polymer.

7. The device of any of the preceding claims further comprising a sample receiving well in fluid communication with said reaction chamber.

8. The device of Claim 7 further comprising a plurality of individual and separate reaction chambers in fluid communication with said sample receiving well.

9. The device of any of the preceding claims further comprising a plurality of individual and separate reaction chambers each with a flow controlling means therein according to the individual assay to be performed.

10. The device of Claims 1-9 wherein the length of said reaction chamber is from 10 to 75 mm.

11. The device of Claims 1-9 wherein said reaction chamber is from 0.01 to 5.0 mm in depth.

12. The device of Claims 1-9 wherein said reaction chamber is from 0.1 to 10.0 mm in width.

13. A method for performing an agglutination assay for detecting the presence or absence of an analyte in a fluid sample, said method comprising providing sufficient reagents to enable said assay to be performed, introducing the fluid sample to be tested into an agglutination reaction device of Claim 1 containing an agglutination reaction chamber, transporting said fluid sample at a first capillary flow rate along the agglutination reaction chamber wherein said first flow rate is induced by said reaction chamber, further transporting said fluid sample along said reaction chamber at a second reduced capillary flow rate wherein said second flow rate is induced by a flow controlling means comprising water-soluble material disposed in a portion of said reaction chamber distal to that portion into which the sample is introduced, and detecting the presence or absence of agglutinated material in the reaction chamber.

14. The method of Claim 13 wherein said reagents are disposed in the agglutination reaction chamber prior to the introduction of the fluid sample into said chamber.

15. The method of Claim 13 wherein said reagents are mixed with said fluid sample prior to the introduction of said sample into said chamber.

## Patentansprüche

1. Vorrichtung zur Durchführung von Agglutinationsreaktionen, wobei die genannte Vorrichtung eine erste hydrophile Oberfläche und eine dazu parallele zweite Oberfläche umfaßt, worin die genannte erste Oberfläche wenigstens ein Kanalbildungsmittel darin umfaßt, so daß, wenn die genannte erste und zweite Oberfläche miteinander in Kontakt gebracht werden, wenigstens eine Agglutinationsreaktionskammer gebildet wird, um Flüssigkeit durch Kapillarwirkung mit einer durch die Kammer ausgelösten Fließgeschwindigkeit zu transportieren, wobei die genannte Kammer entlang ihrer Länge durch die genannte zweite Oberfläche und das genannte Kanalbildungsmittel festgelegt ist, wenigstens eine der genannten ersten und zweiten Oberfläche wenigstes einen Teil von ausreichender Transparenz umfassen, um ein Ablesen der Ergebnisse einer Agglutinationsreaktion zu ermöglichen, wobei die genannte Agglutinationsreaktionskammer ein Mittel zur Kontrolle der Geschwindigkeit des Flüssigkeitsflusses innerhalb der genannten Kammer umfaßt, die ein wasserlösliches Material umfaßt, das in einen Teil der genannten Kammer, entfernt von dem Flüssigkeitsaufnahmeende, eingetrocknet ist, worin das genannte Kontrollmittel eine zweite verringerte Flüssigkeitsfließgeschwindigkeit auslöst, die ausreicht, um das Auftreten einer Agglutinationsreaktion zu ermöglichen.

2. Vorrichtung nach Anspruch 1, worin das genannte wasserlösliche Material Polyvinylpyrrolidon, Polyvinylalkohol, Gelatine oder Rinderserum-Albumin ist.

3. Vorrichtung nach Anspruch 1 oder 2, das außerdem eine zuvor bestimmte Menge eines Reagens zur Durchführung eines Tests umfaßt, die innerhalb der genannten Reaktionskammer und vor dem genannten wasserlöslichen Material, das die Geschwindigkeit des Flüssigkeitsflusses kontrolliert, angeordnet ist.

4. Vorrichtung nach Anspruch 3, worin das genannte Reagens in Form eingetrockneter Flecken oder Streifen angeordnet ist.

5. Vorrichtung nach Anspruch 3, worin das genannten Reagens Mikroteilchen umfaßt.

6. Vorrichtung nach Anspruch 3, worin das genannte Reagens in einem wasserlöslichen Polymeren suspendiert ist.

7. Vorrichtung nach einem der vorgenannten Ansprüche, die außerdem eine Probenaufnahmevertiefung umfaßt, die über die Flüssigkeit mit der genannten Reaktionskammer kommuniziert.

8. Vorrichtung nach Anspruch 7, die ferner eine Vielzahl von einzelnen und getrennten Reaktionskammern umfaßt, die über die Flüssigkeit mit der genannten Probenaufnahmevertiefung kommunizieren.

9. Vorrichtung nach einem der vorgenannten Ansprüche, die außerdem eine Vielzahl von einzelnen und getrennten Reaktionskammern, jeweils mit einem Flußkontrollmittel darin, in Abhängigkeit von den einzelnen durchzuführenden Test, umfaßt.

10. Vorrichtung nach den Ansprüchen 1 bis 9, worin die Länge der genannten Reaktionskammer 10 bis 75 mm beträgt.

11. Vorrichtung nach den Ansprüchen 1 bis 9, worin die genannte Reaktionskammer 0,01 bis 5,0 mm tief ist.

12. Vorrichtung nach den Ansprüchen 1 bis 9, worin die genannte Reaktionskammer 0,1 bis 10,0 mm breit ist.

13. Verfahren zur Durchführung eines Agglutinationstests zum Nachweis des Vorliegens oder der Abwesenheit eines Analyten in einer Flüssigkeitsprobe, wobei das genannte Verfahren umfaßt, Bereitstellen von genügend Reagentien, um eine Durchführung des genannten Tests zu ermöglichen, Einbringen der flüssigen zu testenden Probe in eine Agglutinationsreaktionsvorrichtung nach Anspruch 1, die eine Agglutinationsreaktionskammer enthält, Transportieren der genannten Flüssigkeitsprobe mit einer ersten Kapillarfließgeschwindigkeit entlang der Agglutinationsreaktionskammer, worin die erste genannte Fließgeschwindigkeit durch die genannte Reaktionskammer ausgelöst wird, außerdem Transportieren der genannten Flüssigkeitsprobe entlang der genannten Reaktionskammer mit einer zweiten verringerten Kapillarfließgeschwindigkeit, worin die genannte zweite Fließgeschwindigkeit durch ein Mittel zur Flußkontrolle ausgelöst wird, das ein wasserlösliches Material umfaßt, das in einen Teil der genannten Reaktionskammer, entfernt von dem Teil angeordnet ist, in den die Probe eingebracht wird, und Nachweisen des Vorliegens oder der Abwesenheit von agglutiniertem Material in der Reaktionskammer.

14. Verfahren nach Anspruch 13, bei dem die genannten Reagentien vor dem Einbringen der Flüssigkeitsprobe in die genannte Kammer in der Agglutinationsreaktionskammer angeordnet werden.

15. Verfahren nach Anspruch 13, bei dem die genannten Reagentien mit der genannten Flüssigkeitsprobe vor dem Einbringen der genannten Probe in die genannte Kammer vermischt werden.

## Revendications

1. Dispositif pour réaliser des réactions d'agglutination, ledit dispositif comprenant une première surface hydrophile et une seconde surface parallèle, dans lequel ladite première surface comprend au moins un moyen formant un canal tel que, quand lesdites première et seconde surfaces sont mises en contact l'une avec l'autre il se forme au moins une chambre de réaction d'agglutination pour conduire le fluide par une action capillaire à un débit d'écoulement induit par la chambre, ladite chambre étant définie le long de sa longueur par ladite seconde surface et ledit moyen formant un canal, au moins une desdites première et seconde surfaces comprenant au moins une partie à transparence suffisante pour permettre de détecter les résultats d'une réaction d'agglutination, ladite chambre de réaction d'agglutination comprenant un moyen de contrôle du débit de l'écoulement du fluide dans ladite chambre comprenant un matériau soluble dans l'eau séché dans une portion de ladite chambre distale par rapport a l'extrémité recevant le fluide, dans lequel ledit moyen de contrôle induit un second débit d'écoulement de fluide réduit, suffisant pour permettre à la réaction d'agglutination d'avoir lieu.

2. Dispositif de la revendication 1, dans lequel ledit matériau soluble dans l'eau est la polyvinylpyrrolidone, l'alcool polyvinylique, la gélatine ou la sérum albumine bovine.

3. Dispositif de la revendication 1 ou 2, comprenant en outre une quantité prédéterminée de réactif pour réaliser une analyse, disposé dans ladite chambre de réaction et avant ledit matériau soluble dans l'eau qui contrôle le débit de l'écoulement du fluide.

4. Dispositif de la revendication 3, dans lequel ledit réactif est disposé sous forme de bandelettes ou tâches sèches.

5. Dispositif de la revendication 3, dans lequel ledit réactif contient des microparticules.

6. Dispositif de la revendication 3, dans lequel ledit réactif est en suspension dans un polymère soluble dans l'eau.

7. Dispositif d'une quelconque des revendications qui précèdent, comprenant en outre un puits recevant l'échantillon dont le contenu liquide est en communication avec ladite chambre de réaction.

8. Dispositif de la revendication 7, comprenant en outre une pluralité de chambres de réaction individuelles et séparées dont le contenu liquide est en communication avec ledit puits recevant l'échantillon.

9. Dispositif d'une quelconque des revendications qui précèdent comprenant en outre une pluralité de chambres de réaction individuelles et séparées qui ont chacune un moyen de contrôle de l'écoulement en fonction de l'analyse particulière qui doit y être réalisée.

10. Dispositif des revendications 1 à 9, dans lequel la longueur de ladite chambre de réaction va de 10 à 75 mm.

11. Dispositif des revendications 1 à 9, dans lequel la profondeur de ladite chambre de réaction va de 0,01 à 5,0 mm.

12. Dispositif des revendications 1 à 9, dans lequel la largeur de ladite chambre de réaction va de 0,1 à 10,0 mm.

13. Méthode pour la mise en oeuvre d'une analyse par agglutination pour la détection de la présence ou de l'absence d'un analyte dans un échantillon fluide, ladite méthode comprenant suffisamment de réactifs pour permettre la réalisation de ladite analyse, introduisant l'échantillon fluide à analyser dans un dispositif de réaction d'agglutination de la revendication 1 contenant une chambre de réaction d'agglutination, transportant ledit échantillon fluide avec un premier débit d'écoulement capillaire le long d'une chambre de réaction d'agglutination dans laquelle ledit premier débit d'écoulement est induit par ladite première chambre de réaction, transportant ensuite ledit échantillon de fluide, avec un second débit d'écoulement capillaire réduit, le long de ladite chambre de réaction, dans laquelle ledit second débit d'écoulement est induit par un moyen de contrôle de l'écoulement comprenant un matériau soluble dans l'eau disposé dans une partie de ladite chambre de réaction distale par rapport à la partie dans laquelle l'échantillon est introduit, et détectant la présence ou l'absence de matériau agglutiné dans la chambre de réaction.

14. Méthode de la revendication 13, dans laquelle lesdits réactifs sont disposés dans la chambre de réaction d'agglutination avant l'introduction de l'échantillon de fluide dans ladite chambre.

15. Méthode de la revendication 13, dans laquelle lesdits réactifs sont mélangés audit échantillon de fluide avant l'introduction dudit échantillon dans ladite chambre.
